Europäisches Patentamt

European Patent Office

Office européen des brevets

(19)

(11) Veröffentlichungsnummer: **0 068 048**
**B1**

(12) # EUROPÄISCHE PATENTSCHRIFT

(45) Veröffentlichungstag der Patentschrift:
**19.06.85**

(21) Anmeldenummer: **81110616.0**

(22) Anmeldetag: **18.12.81**

(51) Int. Cl.⁴: **A 61 L 15/04**, A 61 L 15/03,
A 61 K 37/54, A 61 K 37/475,
A 61 K 37/02, A 61 K 35/16

(54) **Angereichertes Plasmaderivat zur Unterstützung von Wundverschluss und Wundabdeckung.**

(30) Priorität: **25.06.81 DE 3124962**

(43) Veröffentlichungstag der Anmeldung:
**05.01.83 Patentblatt 83/1**

(45) Bekanntmachung des Hinweises auf die Patenterteilung:
**19.06.85 Patentblatt 85/25**

(84) Benannte Vertragsstaaten:
**AT BE CH DE FR GB IT LI LU NL SE**

(56) Entgegenhaltungen:
FR - A - 2 448 900

**ARCHIV FÜR ORTHOPÄDISCHE UND
UNFALL-CHIRURGIE, Band 90, Nr. 1, 1977, Seiten 63-65,
Bergman-Verlag, Wiesbaden, DE. P. BÖSCH et al.: "Die
Technik der Fibrinspongiosaplastik"**

(73) Patentinhaber: **Serapharm GmbH & Co. KG,
Kaiser-Wilhelm-Ring 36, D-4400 Münster (DE)**

(72) Erfinder: **Stroetmann, Michael, Kaiser-Wilhelm-Ring 36,
D-4400 Münster (DE)**

(74) Vertreter: **Brehm, Hans-Peter, Dr. Dipl.-Chem. et al,
Patentanwälte Tischer, Kern & Brehm
Albert-Rosshaupter-Strasse 65, D-8000 München 70 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein angereichertes Plasmaderivat in Form eines biochemischen Substrates zur akzelerierten Haemostase und optimalen Regelung des Wundverschlusses. Insbesondere betrifft die Erfindung ein solches Plasmaderivat zur Unterstützung von Wundverschluß und Wundabdeckung auf der Basis von Humanplasmaderivaten mit einem Gehalt an Thrombin und Fibrinolyse-Inhibitor.

Die Aufgabe des Blutgerinnungssystems besteht darin, aus bestimmten Plasmateilen, insbesondere aus gelöstem Fibrinogen, unlösliches Fibrin zu bilden, und dieses auf der Wunde abzulagern, um chemisch und mechanisch die Blutung zum Stillstand zu bringen. Im Verlauf dieser Haemostase bildet Fibrin einen mechanisch-widerstandsfähigen Verschluß von Gewebe- und Gefäßverletzungen. Andererseits ist Fibrin in Verbindung mit dem fibrinolytischen System und den Komponenten des Gerinnungssystems die Grundlage für die zelluläre »Reparatur« von Gewebedefekten.

Wie Forschungsergebnisse in den letzten 10 Jahren gezeigt haben, handelt es sich bei der biochemischen Regelung des Wundverschlusses um einen vielstufigen katalytischen Prozeß, bei dem eine Vielzahl von Vorwärts- und Rückwärtskopplungen involviert sind. Hierbei kommt es zu einem geregelten Zusammenspiel der Blutgerinnungsfaktoren, von denen bislang wenigstens 13 erkannt und charakterisiert worden sind. Der Gerinnungsvorgang selbst findet intravasal Unterstützung durch das Endothel. Zusätzlich kommt den Faktoren der Thrombozyten eine besondere Bedeutung zu.

Die analytische Klärung der Mechanismen der Blutgerinnung, die Isolation der an der Blutgerinnung beteiligten Faktoren und Substanzen, Benutzung passender Ergänzungsmaterialien und die Entwicklung zweckmäßiger Anwendungsverfahren eröffnen der medizinischen Wundbehandlung die Chance einer Akzeleration der Haemostase sowie der optimalen Regelung des Wundverschlusses. Insbesondere wurde es möglich, aus natürlichem Plasma bestimmte Präparate abzutrennen, zu lagern und bei Bedarf zusammen mit weiteren, zur Fibrinbildung und -Vernetzung notwendigen Substanzen auf die Wunde aufzubringen, um eine gezielte Haemostase herbeizuführen.

Ein typisches System dieser Art ist in der Fachwelt unter der Bezeichnung »Fibrinkleber« bekannt. Hierbei wird zunächst Fibrinogenlösung auf die zu adaptierenden Gewebeteile aufgebracht. Sodann werden zur Gerinnung geringe Mengen einer hochkonzentrierten Thrombin- und Faktor XIII-Lösung aufgetropft. Ein Fibrinolyse-Inhibitor wird lokal zugesetzt, um eine vorzeitige Lyse des Fibrins und damit die vorzeitige Dehiszenz der adaptierten Gewebeteile zu vermeiden. Die getrennte Herstellung, Lagerung und Anwendung der genannten Substanzen macht diese Technik aufwendig und umständlich. Darüber hinaus wird gegenüber dem Anforderungsprofil — akzelerierte Haemostase und optimierter Wundverschluß — nur mit einem restringierten Leistungsspektrum (Fibrinogen, Thrombin, Faktor XIII, Fibrinolyse-Inhibitor) gearbeitet.

In der Praxis taut man die tiefgefrorene Fibrinogenlösung auf, versetzt mit Thrombin- und Calciumchlorid, hält das Gemisch eine zeitlang, bis sich die einsetzende Polymerisationsreaktion durch eine Viskositätssteigerung bemerkbar macht, und bringt daraufhin dieses reagierende Gemisch auf den zu verbindenden Gewebeteilen auf. Der Aufwand zur Bereitung des einsatzfähigen »Fibrinklebers« und die geringe Lebensdauer des einsatzbereiten Präparates haben sich in vielen Fällen als hinderlich erwiesen. So ist die Handhabung für den praktizierenden Arzt schwierig, weil er das kurzzeitige Intervall eines noch flüssigen, einsatzbereiten Klebers nicht sicher erfassen kann. Aus der begrenzten Lebensdauer des einsatzbereiten »Fibrinklebers« ergeben sich insbesondere dann Schwierigkeiten, wenn großflächige Abdeckungen von stark blutenden Wunden, gegebenenfalls noch in schlecht zugänglichen Körperhöhlen, erforderlich sind.

Davon ausgehend besteht die Aufgabe der vorliegenden Erfindung darin, ein angereichertes Plasmaderivat zur Unterstützung von Wundverschluß und Wundabdeckung bereitzustellen, das praktisch unbeschränkt bei Raumtemperatur lagerfähig ist, unmittelbar und ohne die Zugabe anderer notwendiger Komponenten auf die Wunde bzw. das Operationsgebiet aufbringbar ist, und das sich hervorragend zum Einsatz in schlecht zugänglichen Körperhöhlen und/oder zur großflächigen Abdeckung von stark blutenden Wunden eignet.

Die erfindungsgemäße Lösung dieser Aufgabe ist ein angereichertes Plasmaderivat in der Form eines biochemischen Substrates zur akzelerierten Haemostase und optimalen Regelung des Wundverschlusses, dessen Zusammensetzung im Hinblick auf eine optimierte Aktivierung des exogenen und/oder endogenen Gerinnungssystems und unter Berücksichtigung einer Vielzahl physiologisch und gegebenenfalls pathologischen Gesichtspunkten ausgewählt ist, zu dessen Hauptbestandteilen Thrombin, das teilweise durch Komponenten des Prothrombinkomplexes ersetzt sein kann, Fibrinolyse-Inhibitoren und wenigstens ein Trockenhalte- und Stabilisierungsmittel aus der Albumin, Globulin und Fibrinogen umfassenden Gruppe gehören und das gegebenenfalls zusätzlich Beimischungen von Blutplättchenextrakten, Antibiotika und dgl. enthalten kann, dessen Bestandteile ausnahmslos in pulverförmigem Zustand vorliegen und das als Spray konfektioniert ist.

Insbesondere sieht die vorliegende Erfindung zur Lösung obiger Aufgabe ein angereichertes Plasmaderivat zur Unterstützung von Wundverschluß und Wundabdeckung auf der Basis von Humanplasmaderivaten mit einem Gehalt an Thrombin und Fibrinolyse-Inhibitor vor, bei dem in einem niedrig siedenden, wasserfreien, als Treibmittel dienenden Lösungsmittel ein Pulvergemisch, enthaltend 15 bis 60 Gew.-% Thrombin, 5 bis 80 Gew.-% Trockenhalte- und Stabilisierungsmittel, nämlich Albumin,

**0 068 048**

Globulin und/oder Fibrinogen und 1 bis 10 Gew.-% Fibrinolyse-Inhibitor (die Prozentangaben beziehen sich jeweils auf das Gesamtgewicht des Pulvergemisches) suspendiert ist. Zum Wundverschluß bzw. zur Wundabdeckung wird ein Sprühstrahl dieser Suspension unter Verdampfung des Lösungsmittels auf die Wunde gerichtet, so daß im wesentlichen nur das trockene, feste Pulvergemisch auf die Wunde gelangt.

Vorteilhafte Ausgestaltungen und Weiterbildungen der Erfindung ergeben sich aus den Unteransprüchen. Diese betreffen vor allem weitere Zusätze zum Pulvergemisch wie Prothrombin, pulverförmiges Kollagen, Plättchenkonzentrat, Faktoren zur Haemophiliebehandlung und Antibiotika und ferner die Auswahl der als Treibmittel dienenden Lösemittel.

Abweichend von den bisher üblichen »Fibrinklebern« beruht die Erfindung auf der Erfahrung, daß zur Unterstützung von Wundverschluß und Wundabdeckung ein zusätzliches Fibrinogen-Angebot nicht in jedem Falle erforderlich ist, und das bekanntlich recht empfindliche Thrombin in festem, trockenem, pulverförmigem Zustand im Gemisch mit ausgewählten feinen, trockenen, pulverförmigen Trockenhalte- und Stabilisierungsmitteln bei Raumtemperatur im wesentlichen unbegrenzt lagerbar ist, ohne daß es zu einer wesentlichen Abnahme seiner biochemischen Aktivität kommt. Selbst wenn das Pulvergemisch als Trockenhalte- und Stabilisierungsmittel festes, trockenes, pulverförmiges Fibrinogen enthält, kommt es wegen des festen Zustandes aller Pulvergemischkomponenten und der Abwesenheit von Wasser auch nach längerer Lagerung bei Raumtemperatur nicht zu einer Fibrinbildung oder einer nennenswerten Abnahme der Aktivität der Gerinnungsenzyme. Die zusätzliche Anwesenheit von Prothrombin steigert die Lagerfähigkeit weiter, insbesondere bei mäßig erhöhten Temperaturen bis maximal etwa 40° C. Andererseits setzt jedoch die biologische Aktivität bei der Gewebeverklebung sowie die Umsetzung zu einer die Blutung stillenden Fibrin-Wundauflage in kurzer Zeitspanne ein, nachdem das trockene Pulvergemisch in der Körperflüssigkeit an- und aufgelöst worden ist. Sehr rasch, beispielsweise nach 2 min, tritt eine akzelerierte Haemostase ein. Die biochemische Regelung des Wundverschlusses wird durch das erhöhte Angebot an Gerinnungsenzymen, vor allem Thrombin und gegebenenfalls Prothrombin, verstärkt und optimiert. Der Zusatz von Fibrinolyse-Inhibitor verhindert die Wiederauflösung des bereits gebildeten Fibringerinnsels. Die Zugabe von Plättchenfaktoren stimuliert die Gerinnung des ausströmenden Blutes, die enthaltenden Wachstumsfaktoren optimieren die Wundbehandlung. Das gegebenenfalls zusätzlich zugesetzte Kollagen absorbiert das Fibringerinnsel und verstärkt die Haftung des Wundverschlußmaterials.

Ausgehend von der Erkenntnis, daß die notwendigen Faktoren trocken miteinander vermischt ohne nennenswerten Aktivitätsverlust bei Raumtemperatur lagerbar sind, wurde nach einem weiteren Gesichtspunkt der Erfindung erkannt, daß dieses trockene Pulvergemisch mit einem geeigneten, das Pulvergemisch nicht anlösenden Treibmittel versprüht werden kann, so daß die Aufbringung des Materials in schlecht zugänglichen Körperhöhlen und/oder die großflächige Abdeckung stark blutender Wunden in kurzer Zeit möglich wird. Voraussetzung ist ein absolut wasserfreies Lösungsmittel, um eine Aktivierung der Pulvergemisch-Komponenten während der Lagerung auszuschließen. Bei Anwendung eines niedrig siedenden Treibmittels wie etwa Frigen® 114 wird erreicht, daß praktisch das gesamte Treibmittel während des Sprühvorganges verdampft, so daß im wesentlichen nur das trockene, feste Pulvergemisch auf die Wunde gelangt.

Die Erfindung schafft somit ein Wundverschluß- und Wundabdeckmaterial, das praktisch unbeschränkt bei Raumtemperatur lagerfähig ist, das unmittelbar und ohne die Zugabe anderer notwendiger Komponenten auf die Wunde bzw. das Operationsgebiet aufbringbar ist, und das vorzüglich zum Einsatz in schlecht zugänglichen Körperhöhlen und/oder zur großflächigen Abdeckung von stark blutenden Wunden geeignet ist. Das auf die Wunde gelangende trockene, feste Pulvergemisch saugt dort Flüssigkeit auf, trocknet die Wundfläche, läßt das Blut gerinnen und unterstützt die Wundheilung. Das Präparat eignet sich deshalb hervorragend zur Behandlung stark nässender und/oder chronisch ulzerierender Wunden. Die Eignung zur Haemophiliebehandlung kann zusätzlich durch die Zugabe von Faktoren VIII und/oder IX verbessert werden.

Besonders bevorzugte Einsatzgebiete für das erfindungsgemäße Wundverschluß- und Wundabdeckmaterial bestehen in der plastischen Chirurgie zum Verkleben und Vernähen von Hautlappen, in der Zahn- und Kiefer-Chirurgie, beispielsweise zum Verschließen von Cavitäten, bei Hals-, Nasen- und Ohreneingriffen, in der Mamma-Chirurgie zum Fixieren von Hautlappen und Gewebeteilen, im Unterbauchbereich, im Vaginalbereich und anderen schwer zugänglichen Körperhöhlen.

Die Erfindung wird nachstehend im einzelnen anhand bevorzugter Ausführungsformen erläutert.

Das mit dem Sprühstrahl versprühte trockene, feste Pulvergemisch muß wenigstens Thrombin, Fibrinolyse-Inhibitor und ein Trockenhalte- und Stabilisierungsmittel nämlich wenigstens einen Bestandteil aus der Albumin, Globulin und Fibrinogen umfassenden Gruppe von Plasmaderivaten enthalten.

Biologisch aktives Thrombin dient als Startsubstanz für die Fibrinbildung und verkürzt die Reaktionszeit der Fibrinogenumwandlung im ausströmenden Blut. Biologisch aktives Thrombin im Sinne dieser Unterlagen liegt dann vor, wenn seine Aktivität unter bekannten, standardisierten Bedingungen wenigstens 1000 internationale Einheiten pro mg Thrombin beträgt. Geeignete Präparate sind handelsüblich zugänglich. Beispielsweise kann geeignetes Thrombin in mikrokristalliner Form mit einer biologischen Aktivität von wenigstens 3000 Einheiten pro mg Thrombin unter der Handelsbezeichnung

3

»Topostasin« von Hoffmann LaRoche, Grenzach, Baden bezogen werden.

Nach einem wesentlichen Gesichtspunkt der Erfindung ist ein überdurchschnittlich hohes Thrombinangebot vorgesehen. Deshalb soll der Thrombingehalt im Pulvergemisch wenigstens 15 Gew.-% betragen. Die Obergrenze des Thrombingehaltes hängt von der Wirksamkeit des Trockenhalte- und Stabilisierungsmittel ab und kann bis zu 60 Gew.-% betragen. Gute Ergebnisse wurden mit einem Thrombingehalt von 20 bis 50 Gew.-% erzielt; besonders bevorzugt wird ein Thrombingehalt des trockenen Pulvergemisches von etwa 40 bis 45 Gew.-%.

Ein Teil des Thrombins kann durch Prothrombin ersetzt sein. Das Gerinnungsenzym Thrombin ist empfindlich, und seine biologische Aktivität nimmt bei längerer Lagerung ab. Demgegenüber bildet Prothrombin eine stabile, über lange Zeiträume lagerfähige Thrombinreserve, die bei Feuchtigkeitszutritt durch vorhandenes Thrombin und/oder das anströmende Blut aktiviert wird.

Das feste Pulvergemisch kann auf 1 Gew.-Teil Thrombin 0,1 bis 2 Gew.-Teile Prothrombin enthalten. Vorzugsweise sind auf 1 Gew.-Teil Thrombin 0,5 bis 0,9 Gew.-Teile Prothrombin vorgesehen. Ein vergleichsweise hoher Prothrombingehalt ist zweckmäßig, da Prothrombin zumeist in einem Faktorenkomplex vorliegt, der weitere Gerinnungsenzyme enthält, so daß ein hoher Prothrombingehalt auch einen hohen Anteil an diesen Gerinnungsenzymen bewirkt, das wiederum die Blutgerinnung beschleunigt. Der Prothrombingehalt kann deshalb 5 bis 40 Gew.-% vorzugsweise 20 bis 35 Gew.-% ausmachen. Prothrombin kann aus käuflichem Prothrombinkomplex abgetrennt oder vom Plasma durch Bariumsulfat extrahiert und aus dem kristallinen Niederschlag rückgewonnen werden. Daneben ist Prothrombin auch handelsüblich zugänglich, beispielsweise als PPSB-Präparat von der Firma Imuno AG, Wien. Gut geeignet sind solche Prothrombinpräparate, die beim Einbringen in Körperflüssigkeit zu wenigstens 95% in Thrombin umwandelbar sind.

Als weiteren notwendigen Bestandteil enthält das trockene, feste Pulvergemisch 1 bis 10 Gew.-%, vorzugsweise 2 bis 6 Gew.-% Fibrinolyse-Inhibitor. Geeignete Fibrinolyse-Inhibitoren sind bekannt. Vorzugsweise dient als Fibrinolyse-Inhibitor ein oder mehrere Antiplasmine. Beispielhafte Antiplasmine sind Aprothenin, $\alpha_1$-Antiplasmin und/oder Trypsininhibitor. Gut geeignet ist auch ein 1 : 1-Gemisch aus $\alpha_1$-Antiplasmin und $\alpha_2$-Makroglobulin. Der Zusatz derartiger Antiplasmine verhindert die Wiederauflösung des bereits gebildeten Fibringerinnsels. Nach einem beispielhaften Verfahren zur Gewinnung von geeignetem $\alpha_1$-Antiplasmin wird Fibrinogen konvalent an Sepherose gebunden und durch Thrombin zu Fibrin umgewandelt. Das so immobilisierte Fibrin dient als Rezeptor für das plasmatische Antiplasmin, das bei Durchlauf von Plasma durch die Säure gebunden wird, und mit $\varepsilon$-Aminocapronsäure ausgewaschen werden kann.

Weiterhin enthält das trockene, feste Pulvergemisch wenigstens ein Trockenhalte- und Stabilisierungsmittel. Aufgabe dieses Trockenhalte- und Stabilisierungsmittels ist es, die Versprühbarkeit und die Lagerfähigkeit des festen Thrombins ohne nennenswerten Aktivitätsverlust in Gegenwart des Treibmittels zu gewährleisten. Als Trockenhalte- und Stabilisierungsmittel ist wenigstens eines der Plasmaderivate Albumin, Globulin oder Fibrinogen vorgesehen. Entgegen den bekannten Vorschlägen muß das erfindungsgemäße Wundverschluß- und Wundabdeckmaterial nicht notwendigerweise Fibrinogen enthalten, da eine befriedigende blutstillende und die Wunde verschließende Wirkung bereits durch das hohe Thrombinangebot, gegebenenfalls verstärkt durch die Anwesenheit von Prothrombin gewährleistet wird. Beispielsweise kann das erfindungsgemäße Material aus den genannten Plasmaderivaten lediglich Albumin enthalten, das dann vor allem als Trockenhalte- und Stabilisierungsmittel wirkt und die Lagerfähigkeit, biologische Aktivität und Versprühbarkeit des festen, pulverförmigen Thrombins gewährleistet. Derart festes, mikrokristallines Albumin ist handelsüblich zugänglich und kann beispielsweise von der Firma Behring-Werke, Marburg, bezogen werden.

Alternativ kann als Plasmaderivat allein Globulin vorgesehen werden. Vorzugsweise handelt es sich um das handelsübliche Gemisch aus $\alpha$-, $\beta$- und $\gamma$-Globulin, wie es beispielsweise von Böhringer, Mannheim vertrieben wird.

Als weiteres Trockenhalte- und Stabilisierungsmittel kommt aus Humanplasma gewonnenes Fibrinogen in Betracht. Geeignete Präparate sind ebenfalls handelsüblich zugänglich, beispielsweise von Behring-Werke, Marburg.

Ferner kann ein gut geeignetes Fibrinogen aus Humanplasma durch Fällung mit einem Glycin, $\beta$-Alanin und Äthanol enthaltendem Lösungsmittelgemisch nach anschließender Dialyse und Lyophilisation des Fällungsproduktes erhalten werden. Derartiges, mikrokristallines Fibrinogen weist ein Molekulargewicht von 340 000 ± 5% auf, ist in der $\alpha$-Kette leicht angedaut, löst sich nach Einbringen in Körperflüssigkeit schnell, wobei der in Lösung gerinnbare Anteil des Fibrinogens wenigstens 85% betragen soll, und beginnt unmittelbar darauf, beispielsweise in weniger als 2 min, zu polymerisieren. 10 Gew.-Teile derartiges Fibrinogen enthalten weniger als 0,1 Gew.-Teil kälteunlösliches Globulin. Es ist festgestellt worden, daß die Fibrinpolymerisation um so schneller verläuft, je weniger kälteunlösliches Globulin vorhanden ist. Sofern das Fibrinogen nicht nur als Trockenhalte- und Stabilisierungsmittel für das Thrombin dient, sondern auch das Fibrinogenangebot im Wundgebiet erhöhen soll, wird daher vorzugsweise dieses, an kälteunlöslichem Globulin verarmte Fibrinogen eingesetzt.

Anstelle einer einzigen Komponente kann als Trockenhalte- und Stabilisierungsmittel auch ein Gemisch von zwei oder mehr Komponenten aus der Albumin, Globulin und Fibrinogen umfassenden Gruppe vorgesehen werden. Gut bewährt hat sich beispielsweise ein Gemisch aus Albumin und Fibrinogen.

Die Summe der Anteile an Albumin, Globulin und/oder Fibrinogen kann 5 bis 80% des Gewichtes des trockenen, festen Pulvergemisches ausmachen. Vorzugsweise ist ein Anteil von etwa 8 bis 70 Gew.-% vorgesehen.

Weiterhin kann das feste Pulvergemisch festes, pulverförmiges, in Wasser lösliches Kollagen enthalten. Geeignetes Kollagen wurde aus Sehnen oder Haut gewonnen, weist im Mittel ein Molekulargewicht von etwa 3 bis 5 Millionen auf; das pulverförmige Kollagen ist wenigstens zu 90% in Wasser löslich.

Das als Bestandteil des Pulvergemisches auf die Wunde geblasene Kollagen saugt Flüssigkeit auf und aktiviert die mit dem ausströmenden Blut herangeführten Thrombozyten, und beschleunigt damit die Blutgerinnung. Ferner erhöht das Kollagen die Viskosität im Wundgebiet und erleichtert die Haftung des Wundverschlußmaterials an den Gewebeteilen. Nach einer bevorzugten Ausführungsform der Erfindung soll das feste Pulvergemisch zusätzlich Kollagen enthalten. Der Kollagenanteil ist nicht kritisch und kann etwa von 2 bis 24% des Pulvergewichtes reichen.

Sofern das Pulvergemisch Kollagen enthält, kann der Anteil an Trockenhalte- und Stabilisierungsmittel, nämlich den Plasmaderivaten — Albumin, Globulin und/oder Fibrinogen — verringert werden. Gut bewährt hat sich in diesem Fall ein Anteil von etwa 3 bis 12 Gew.-% Kollagen und von 8 bis 70 Gew.-% für die Summe der Anteile an Albumin, Globulin und/oder Fibrinogen.

Weiterhin kann das feste Pulvergemisch zusätzlich Plättchenextrakt enthalten. Die Plättchen gehören zu den zellulären Bestandteilen des Blutes. Sie werden durch Zentrifugation von den Erythrozyten als »buffy coat« getrennt. Diese Zellfraktion wird mit zuckerhaltigen Lösungen gewaschen und dabei von noch beigemengten Erythrozyten befreit. Durch geeignete Zellyse werden die Membranen der Zellen freigesetzt und vom Zellsaft durch Zentrifugation abgetrennt. Die Zellwände der Plättchen enthalten Phospholipid-Protein-Strukturen, die sowohl zur Aktivierung der endogenen Gerinnungskaskade als auch zur Orientierung der an der Gerinnung beteiligten Enzyme dienen. Nach Trocknen der Membranpartikel, Homogenisation und Extration wird daraus ein Produkt gewonnen, das unter physiologischen Bedingungen löslich und reaktiv ist.

Im Hinblick auf die hohe Wirksamkeit ist ein geringer Anteil an Plättchenextrakt ausreichend, etwa von 0,2 bis 2% des Gewichts des Pulvergemisches; vorzugsweise soll der Plättchenextrakt-Anteil 0,5 bis 1,2 Gew.-% betragen.

Ein beispielshafter Plättchenextrakt wird wie folgt erhalten:

»Buffy Coat« eines Sedimentes aus humanem Vollblut wird mit Seiler-Lösung (Glucose-Salzgemisch) zur Abtrennung der Erythrozyten erschöpfend gewaschen. Das so vorbereitete Leukozyten-Monozyten-Thrombozyten-Präparat wird durch Zusatz von Triton® X gelöst, der unlösliche Anteil abzentrifugiert und die überstehende Lösung mit gesättigtem Ammoniumsulfat bei einem pH-Wert von 7,4 fraktioniert gefällt. Das Sediment wird abzentrifugiert, dialysiert und getrocknet. Der Phospholipidgehalt der Fraktion beträgt etwa 16 bis 25%. Bei der Prüfung im Thromboplasmintest erweist sich das Präparat als gerinnungsaktiv. Die Wachstumssteigerung wird durch Fibroplastenvermehrung in der Kultur überprüft.

Neben den oben genannten, notwendigen und wahlweise vorgesehenen Komponenten des Pulvergemisches kann dieses weitere, bekannte, auf den Blutgerinnungsvorgang einwirkende sowie die Wundheilung beeinflussende Faktoren und Substanzen, alle in fester pulverförmiger Form, enthalten. Ferner kann das feste Pulvergemisch zusätzlich mit Bakteriziden und/oder Antibiotika angereichert werden und/oder andere zur Bekämpfung bestimmter pathologischer Zustände wirksame Zusätze enthalten; hierzu gehören beispielsweise Antibiotika wie Penicilline, Aureomycine, Streptomycine und dgl., ferner Antihistaminika, Vasopressine und weiterhin die Gerinnungsfaktoren VIII und/oder IX zur haemophilen Wundversorgung. Der Anteil der genannten Antibiotika kann etwa 10 000 bis 50 000 Einheiten auf 1 g Pulvergemisch betragen. Der Anteil der Gerinnungsfaktoren VIII und/oder IX kann etwa 1 bis 10 Einheiten auf 1 g Pulvergemisch betragen. Die Anwesenheit dieser für die Haemophiliebehandlung wichtigen Faktoren VIII und/oder IX vermag nicht nur die Wunde zu verschließen, sondern aktiviert auch das körpereigene Blut zum Wundverschluß. Ferner können die Aktivierung der Gerinnungsenzyme fördernde Salze, wie etwa $CaCl_2$ vorhanden sein.

Eine bevorzugte Ausführungsform der Erfindung sieht für das zu versprühende, feste Pulvergemisch die nachstehende Zusammensetzung vor (die Prozentangaben beziehen sich auf das Gesamtgewicht des Pulvergemisches).

| | |
|---|---|
| 20 bis 50 Gew.-% | Thrombin |
| 5 bis 40 Gew.-% | Prothrombin |
| 8 bis 70 Gew.-% | Albumin, Globulin und/oder Fibrinogen, |
| 2 bis 6 Gew.-% | Fibrinolyse-Inhibitor, |
| 3 bis 12 Gew.-% | wasserlösliches Kollagen, |
| 0,5 bis 1,2 Gew.-% | Plättchenextrakt. |

Zusätzlich kann das Pulvergemisch

10 000 bis 50 000 Einheiten Anitbiotika auf 1 g Pulvergemisch und
1 bis 10 Einheiten Faktor VIII und/oder IX auf 1 g Pulvergemisch

enthalten.

Alle oben genannten Präparate sind bei Raumtemperatur und bei Temperaturen bis zu 56° C fest und im wesentlichen mikrokristallin. Das feste Pulvergemisch wird aus diesen Komponenten durch einfaches, trockenes Vermischen erhalten. Das Vermischen kann beispielsweise durch 10 min lange Behandlung in einer Kugelmühle erfolgen. Alternativ kann zum Vermischen eine Ultrabeschallung und Siebung eingesetzt werden. In jedem Fall erhält man ein trockenes, frei fließendes Pulver aus dem homogenen Gemisch der Bestandteile.

Zur Gewährleistung einer guten Versprühbarkeit soll die mittlere Teilchengröße des Pulvergemisches zwischen etwa 0,1 und etwa 5 µm gehalten werden. Bei noch feineren Pulvern besteht die Gefahr einer Zusammenballung und Klumpenbildung. Gröbere Pulver lassen sich nicht ausreichend versprühen, und ihre Auflösung in der Körperflüssigkeit ist verzögert. In Verbindung mit herkömmlichen Sprühköpfen, die beispielsweise einen Durchmesser von 5 µm aufweisen, hat sich eine mittlere Teilchengröße des Pulvergemisches von etwa 0,5 bis 2 µm gut bewährt; besonders bevorzugt wird in diesem Falle eine mittlere Teilchengröße des Pulvergemisches von etwa 1 bis 1,5 µm. Zur Gewährleistung dieser Teilchengröße wird das Pulver ausreichend fein vermahlen und das Mahlprodukt gesiebt.

Um ein versprühbares Wundverschluß- und Wundabdeckmaterial bereitzustellen, ist das feste Pulvergemisch aus den oben genannten Komponenten in einem niedrig siedenden, wasserfreien, als Treibmittel dienenden Lösungsmittel suspendiert. Ein brauchbares, niedrig siedendes Lösungsmittel liegt dann vor, wenn das Lösungsmittel nach dem Verlassen der Sprühdüse vor Erreichen der Wundoberfläche vollständig verdampft. Auch bei Anwendung physiologisch inerter und unbedenklicher Lösungsmittel wird damit erreicht, daß beim Versprühen im wesentlichen nur das trockene, feste Pulvergemisch auf die Wunde gelangt, während der Lösungsmittelanteil, sofern dieser überhaupt auf die Wunde gelangt, unterhalb der Nachweisgrenze spezifischer Wirkungen bleibt. Um eine ausreichend schnelle Verdampfung zu gewährleisten, soll der Siedepunkt des Lösungsmittels unter Atmosphärendruck (etwa 100 kPa) unter 10° C liegen. Für den erfindungsgemäßen Zweck gut geeignete, bei Raumtemperatur lediglich unter erhöhtem Druck flüssige Lösungsmittel sind einzelne ausgewählte halogenierte Kohlenwasserstoffe oder ein Azeotrop aus solchen Kohlenwasserstoffen, wie sie unter dem eingetragenen Warenzeichen »Frigen« bekannt sind. Gut geeignet ist beispielsweise »Frigen 114« (Tetrafluordichloräthan $C_2F_4Cl_2$, Siedepunkt 4,1° C) oder »Frigen 13« (Chlortrifluormethan $CF_3Cl$, Siedepunkt −81,4° C) oder »Frigen 12« (Difluordichlormethan $CF_2Cl_2$, Siedepunkt −30,0° C). Sofern als Treibmittel derartige Frigene verwendet werden, kommen vorzugsweise auf 1 Gew.-Teil Pulvergemisch 3 bis 6 Gew.-Teile Frigen-Treibmittel. Daneben kommen als Treibmittel verflüssigte Gase wie etwa flüssiges Kohlendioxid, flüssiger Stickstoff, flüssiges Methan, flüssiges Lachgas (Stickoxydul $N_2O$) oder dgl. in Betracht. Auch niedrig siedende Gemische organischer Lösungsmittel wie etwa Aceton/Alkohol-Gemische oder Aceton/Äther-Gemische (beispielsweise 8 Vol.-Teile Aceton auf 2 Vol.-Teile Äther) können in Einzelfällen vorgesehen werden. Das Versprühen solcher niedrig siedender organischer Lösungsmittel wird zusätzlich zum eigenen Dampfdruck vorteilhafterweise zusätzlich durch eine mechanische Volumenverkleinerung des Behälterinnenraums unterstützt, etwa über einen mechanisch oder Feder-betätigten Druckkolben, eine pneumatisch betätigte Membran oder dgl.

Es ist wichtig, daß das als Treibmittel vorgesehene niedrig siedende Lösungsmittel wasserfrei ist, um eine Aktivierung der Gerinnungsenzyme und des gegebenenfalls vorhandenen Fibrinogens im Verlauf der Lagerung sicher auszuschließen. Der von den Herstellern garantierte Feuchtigkeitsgehalt der Frigene von nicht höher als 10 mg/kg (= 0,001%) erweist sich für die vorliegenden Zwecke als ausreichend, so daß zusätzliche Trocknungsmaßnahmen nicht erforderlich sind.

Nachstehend sind in tabellarischer Form beispielhafte Zusammensetzungen des angereicherten Plasmaderivates zur Unterstützung von Wundverschluß und Wundabdeckung angegeben.

Tabelle

| | Beispiele | | | | | | | |
|---|---|---|---|---|---|---|---|---|
| | I | II | III | IV | V | VI | VII | VIII |
| Bestandteile (g) | | | | | | | | |
| Thrombin | 30 | 48 | 40 | 25 | 35 | 45 | 50 | 20 |
| Prothrombin | | | 30 | 35 | 25 | 15 | 34,5 | 5,8 |

Tabelle (Fortsetzung)

| | Beispiele I | II | III | IV | V | VI | VII | VIII |
|---|---|---|---|---|---|---|---|---|
| Fibrinogen | 25 | | 25 | 15 | 29,4 | | | 30 |
| Albumin | 20 | 30 | | 5 | | 29,2 | | 15 |
| Globulin | 20 | 20 | | 5 | | | 10 | 10 |
| Kollagen | | | | 10 | 5 | 7 | 3 | 12 |
| Plättchenextrakt | | | | 1 | 0,6 | 0,8 | 0,5 | 1,2 |
| Fibrinolyse-Inhibitor*) | 5 | 2 | 5 | 4 | 5 | 3 | 2 | 6 |
| | | | | | | | | |
| Treibmittel (g) | | | | | | | | |
| Frigen 114 | 400 | | | 350 | 400 | | 400 | 500 |
| Frigen 12 | | 500 | | | | 500 | | |
| Frigen 13 | | | 600 | | | | | |

*) Als Fibrinolyse-Inhibitor diente in allen Beispielen »Trasylol®«, bezogen von der Fa. Bayer AG, Leverkusen.

Beim Versprühen des pulverförmigen und gerinnungsaktiven Materials entsprechend den obigen Beispielen I bis VIII aus einer Entfernung von etwa 10 bis 30 cm entsteht an der Wundfläche ein dünner weißlicher Film, der sofort von austretendem Blut gelöst wird. In wenigen Minuten ist die eingesprühte Wundfläche durch die Blutgerinnung abgedichtet und verschlossen.

An dem in Beispiel V verwendeten Pulvergemisch wurden die Thrombinaktivität, die Fibrin-Vernetzbarkeit und die Gerinnungsaktivität geprüft. Hierbei wurden die nachstehenden Ergebnisse erhalten.

Das trockene Pulvergemisch gemäß Beispiel V wurde in einer Konzentration von 0,5 mg Pulvergemisch pro 1 ml 0,9%iger, wäßriger NaCl-Lösung gelöst. 100-µl-Proben dieser Lösung wurden an einer Standardlösung eines chromogenen Substrates (S2222 der Firma Kabivitrum, Stockholm) getestet. Bei der Endpunktanalyse muß der Extinktionszuwachs bei 405 nm einer Thrombinaktivität von wenigstens 0,001 internationalen Einheiten entsprechen. Das System wird mit bekannten Thrombinmengen geeicht, so daß es leicht möglich ist, dazwischenliegende Werte zu bestimmen.

Im vorliegenden Falle konnten 0,0025 bis 0,003 Einheiten nachgewiesen werden. Die »Einheiten« haben die Bedeutung, daß 1 Einheit 1 ml einer standardisierten Fibrinogenlösung in 15 sec zum Gerinnen bringen muß.

## Prüfung der Fibrin-Vernetzbarkeit

Die durch Thrombin gebildeten Fibrin-Gerinnsel wurden sofort erschöpfend in 0,9%iger wäßriger NaCl-Lösung gewaschen und daraufhin in 0,1%iger Monochloressigsäure gelöst. Als Bezugsgröße dient der Extinktionswert bei 280 nm. Die in zeitlich definierten Abständen später aus dem Ansatz entfernten Gerinnsel sind weniger löslich.

Ihre Extinktionswerte werden mit dem Nullwert verglichen. Nach 30 min ist das gebildete Fibrin bei 37° C in dem angegebenen Lösungsmittel nicht mehr nachweisbar.

## Prüfung der Gerinnungsaktivität des angereicherten Plasmaderivates

10-mg-Portionen des trockenen Pulvergemisches gemäß Beispiel V wurden in einer 5 mM CaCl₂-enthaltenden 0,9%igen wäßrigen NaCl-Lösung unter Rühren gelöst. Die Gerinnungsaktivität dieser Lösung wurde durch die Geschwindigkeit der Fibrinbildung bestimmt. Dazu wurden in definierten Zeitabständen Proben entnommen und in der Elektrophorese auf die Anteile auf Fibrinogen und Fibrinoligomere untersucht. Die Gerinnungszeit beträgt unter den gewählten Bedingungen 70 bis 90 sec. Dabei werden 35% des Fibrinogens zu Fibrinmonomeren umgewandelt. Die Vernetzung der Fibrinfäden durch den im Präparat enthaltenden Faktor XIII ist innerhalb von 30 min beendet. Danach läßt sich das Präparat in 0,1%iger Monochloressigsäure nicht mehr auflösen.

Die biologische Aktivität bei der Gewebeverklebung sowie die Umsetzung zu einer die Blutung stillenden Fibrin-Wundauflage setzt ein, nachdem das trockene, pulverförmige Plasmaderivat in Körperflüssigkeit an- und aufgelöst ist. Bereits nach kurzer Zeit, beispielsweise nach 2 min, tritt eine akzelerierte Haemostase ein. Die biochemische Regelung des Wundverschlusses wird durch das erhöhte Angebot an Thrombin, Fibrinolyse-Inhibitoren verstärkt und optimiert. Die Zugabe von Plättchenfaktoren stimuliert die Gerinnung des ausströmenden Blutes, die enthaltenden Wachstumsfaktoren optimieren die Wundheilung.

Jede blutende Wunde liefert gerinnbares Material, das wegen der Strömungsgeschwindigkeit von den Wundrändern weggespült wird. Gerinnbare, trockene Wundpuder erhöhen lokal das Gerinnungspotential, saugen Flüssigkeit auf und fördern die Plättchenadhäsion. Das im Wundgebiet exponierte Kollagen adsorbiert das Fibringerinnsel und verstärkt die Haftung des Wundverschlußmaterials. Durch die trockene Applikationsform erübrigt sich eine besondere Aufbewahrung oder eine Vermischung mit Thrombin nach der Anwendung. Das Verkleben von Hautlappen, Sicherung von Operationsnähten oder das Unterbinden von Sickerblutungen wird durch die Sprayform besonders einfach in der Handhabung.

## Patentansprüche

1. Angereichertes Plasmaderivat
in Form eines biochemischen Substrates zur akzelerierten Haemostase und optimierten Regelung des Wundverschlusses, dessen Zusammensetzung im Hinblick auf eine optimierte Aktivierung des exogenen und/oder endogenen Gerinnungssystems unter Berücksichtigung einer Vielzahl physiologischer und gegebenenfalls pathologischer Gesichtspunkte ausgewählt ist,
zu dessen Hauptbestandteilen Thrombin, das teilweise durch Komponenten des Prothrombinkomplexes ersetzt sein kann, Fibrinolyse-Inhibitoren und wenigstens ein Trockenhalte- und Stabilisierungsmittel aus der Albumin, Globulin und Fibrinogen umfassenden Gruppe gehören, und das gegebenenfalls zusätzlich Beimischungen von Blutplättchenextrakten, Antibiotika und dgl. enthalten kann,
dessen Bestandteile ausnahmslos in pulverförmigem Zustand vorliegen, und
das als Spray konfektioniert ist.

2. Angereichertes Plasmaderivat nach Anspruch 1 zur Unterstützung von Wundverschluß und Wundabdeckung auf der Basis von Humanplasmaderivaten mit einem Gehalt an Thrombin und Fibrinolyse-Inhibitor, dadurch gekennzeichnet, daß in einem niedrig siedenden, wasserfreien, als Treibmittel dienenden Lösungsmittel ein Pulvergemisch, enthaltend

| | |
|---|---|
| 15 bis 60 Gew.-% | Thrombin, |
| 5 bis 80 Gew.-% | Trockenhalte- und Stabilisierungsmittel, nämlich Albumin, Globulin und/oder Fibrinogen, und |
| 1 bis 10 Gew.-% | Fibrinolyse-Inhibitor |

(jeweils bezogen auf das Pulvergewicht) suspendiert ist.

3. Plasmaderivat nach Anspruch 2, dadurch gekennzeichnet, daß ein Teil des Thrombins durch Prothrombin ersetzt ist.

4. Plasmaderivat nach Anspruch 3, dadurch gekennzeichnet, daß das Pulvergemisch 5 bis 40 Gew.-% Prothrombin enthält.

5. Plasmaderivat nach einem der Ansprüche 2 bis 4, dadurch gekennzeichnet, daß das Pulvergemisch zusätzlich 2 bis 24 Gew.-% pulverförmiges, in Wasser lösliches Kollagen enthält.

6. Plasmaderivat nach einem der Ansprüche 2 bis 5, dadurch gekennzeichnet, daß das Pulvergemisch zusätzlich 0,2 bis 2 Gew.-% Plättchenextrakt enthält.

7. Plasmaderivat nach einem der Ansprüche 2 bis 6, dadurch gekennzeichnet, daß das Pulvergemisch besteht aus

| | |
|---|---|
| 20 bis 50 Gew.-% | Thrombin, |
| 5 bis 40 Gew.-% | Prothrombin, |
| 8 bis 70 Gew.-% | Albumin, Globulin und/oder Fibrinogen, |
| 3 bis 12 Gew.-% | wasserlösliches Kollagen, |
| 0,5 bis 1,2 Gew.-% | Plättchenextrakt und |
| 2 bis 6 Gew.-% | Fibrinolyse-Inhibitor. |

8. Plasmaderivat nach einem der Ansprüche 2 bis 7, dadurch gekennzeichnet, daß das Pulvergemisch zusätzlich mit 1 bis 10 Einheiten Faktor VIII und/oder Faktor IX pro 1 g Pulvergemisch angereichert ist.

0 068 048

9. Plasmaderivat nach einem der Ansprüche 2 bis 8, dadurch gekennzeichnet, daß das Pulvergemisch zusätzlich mit Bakteriziden und/oder Antibiotika, wie Penicilline, Aureomycine, Streptomycine und dgl. angereichert ist.

10. Plasmaderivat nach einem der Ansprüche 2 bis 9, dadurch gekennzeichnet, daß das Pulvergemisch eine mittlere Teilchengröße zwischen 0,1 und 5 μm aufweist.

11. Plasmaderivat nach einem der Ansprüche 2 bis 10, dadurch gekennzeichnet, daß das als Treibmittel dienende Lösungsmittel bei Atmosphärendruck (100 kPa) unterhalb 10° C siedet.

12. Plasmaderivat nach einem der Ansprüche 1 bis 11, dadurch gekennzeichnet, daß das als Treibmittel dienende Lösungsmittel ein aliphatischer, halogenierter Kohlenwasserstoff ist.

13. Plasmaderivat nach Anspruch 12, dadurch gekennzeichnet, daß der aliphatische, halogenierte Kohlenwasserstoff Tetrafluor-dichloräthan ($C_2F_4Cl_2$) oder Difluordichlormethan ($CF_2Cl_2$) ist.

14. Verfahren zur Herstellung eines angereicherten Plasmaderivates
in Form eines biochemischen Substrates zur akzelerierten Haemostase und optimierten Regelung des Wundverschlusses, dessen Zusammensetzung im Hinblick auf eine optimierte Aktivierung des exogenen und/oder endogenen Gerinnungssystems unter Berücksichtigung einer Vielzahl physiologischer und gegebenenfalls pathologischer Gesichtspunkte ausgewählt wird,
dadurch gekennzeichnet, daß
die ausnahmslos in pulverförmigem Zustand vorliegenden Bestandteile, nämlich Thrombin, das teilweise durch Komponenten des Prothrombinkomplexes ersetzt sein kann, Fibrinolyse-Inhibitoren und wenigstens ein Trockenhalte- und Stabilisierungsmittel aus der Albumin, Globulin und Fibrinogen umfassenden Gruppe, sowie gegebenenfalls vorhandene Beimischungen von Blutplättchenextrakten, Antibiotika und dergleichen zusammen mit einem Treibmittel als Spray konfektioniert werden.

15. Verfahren nach Anspruch 14, dadurch gekennzeichnet, daß in einem niedrigsiedenden, wasserfreien, als Treibmittel dienenden Lösungsmittel ein Pulvergemisch, enthaltend

| 15 bis 60 Gew.-% | Thrombin, |
| 5 bis 80 Gew.-% | Trockenhalte- und Stabilisierungsmittel, nämlich Albumin, Globulin und/oder Fibrinogen, und |
| 1 bis 10 Gew.-% | Fibrinolyse-Inhibitor |

(jeweils bezogen auf das Pulvergemisch) suspendiert wird.

16. Verfahren nach Anspruch 15, dadurch gekennzeichnet, daß ein Teil des Thrombins durch Prothrombin ersetzt wird.

17. Verfahren nach Anspruch 15 oder 16, dadurch gekennzeichnet, daß dem Pulvergemisch zusätzlich 2 bis 24 Gew.-% pulverförmiges, in Wasser lösliches Kollagen zugesetzt wird.

18. Verfahren nach einem der Ansprüche 15 bis 17, dadurch gekennzeichnet, daß dem Pulvergemisch zusätzlich 0,2 bis 2 Gew.-% Plättchenextrakt zugesetzt wird.

19. Verfahren nach einem der Ansprüche 15 bis 18, dadurch gekennzeichnet, daß ein Pulvergemisch aus

| 20 bis 50 Gew.-% | Thrombin, |
| 5 bis 40 Gew.-% | Prothrombin, |
| 8 bis 70 Gew.-% | Albumin, Globulin und/oder Fibrinogen, |
| 3 bis 12 Gew.-% | wasserlösliches Kollagen, |
| 0,5 bis 1,2 Gew.-% | Plättchenextrakt und |
| 2 bis 6 Gew.-% | Fibrinolyse-Inhibitor |

in dem Treibmittel suspendiert wird.

20. Verfahren nach einem der Ansprüche 15 bis 19, dadurch gekennzeichnet, daß das Pulvergemisch zusätzlich mit 1 bis 10 Einheiten Faktor VIII und/oder Faktor IX pro 1 g Pulvergemisch angereichert wird und/oder zusätzlich mit Bakteriziden und/oder Antibiotika, wie Penicilline, Aureomycine, Streptomycine und dergleichen angereichert wird.

21. Verfahren nach einem der Ansprüche 14 bis 20, dadurch gekennzeichnet, daß als Treibmittel ein aliphatischer, halogenierter Kohlenwasserstoff dient.

22. Verfahren nach Anspruch 21, dadurch gekennzeichnet, daß als aliphatischer, halogenierter Kohlenwasserstoff Tetrafluor-dichloräthan ($C_2F_4Cl_2$) oder Difluor-dichlormethan ($CF_2Cl_2$) dient.

## Claims

1. An enriched plasma derivative
in form of a biochemical substrate useful for accelerated hemostasis and optimized control of wound sealing, the composition thereof is selected with regard to an optimized activation of the exogenic and/or endogenic coagulation system and under consideration of a multiplicity of physiological and, if applicable, pathological aspects,

9

the main constituents thereof include thrombin which may partly be replaced by components of the prothrombin complex, fibrinolysis inhibitors and at least one desiccating and stabilizing agent selected from a group including albumin, globulin and fibrinogen, and which may optionally contain additional admixtures of blood platelet extracts, antibiotics, and the like, the constituents exclusively are provided in powdery state, and which is prepared in the form of a spray.

2. The enriched plasma derivative according to claim 1, for enhancement of wound sealing and wound convering, based on human plasma derivatives containing thrombin and a fibrinolysis inhibitor, characterized in that a powdery admixture containing

| 15 to 60% | by weight of thrombin, |
| 5 to 80% | by weight of at least one desiccating and stabilizing agent selected from albumin, globulin, and fibrinogen, and |
| 1 to 10% | by weight of a fibrinolysis inhibitor |

(respectively based on the weight of the powder) is suspended in a low-boiling anhydrous solvent serving as propellant.

3. The plasma derivative according to claim 2, wherein a part of the thrombin is replaced by prothrombin.

4. The plasma derivative according to claim 3, wherein the powdery mixture contains 5 to 40% by weight of prothrombin.

5. The plasma derivative according to anyone of the claims 2 to 4, wherein the powdery mixture additionally contains 2 to 24% by weight of powdery, water-soluble collagen.

6. The plasma derivative according to anyone of the claims 2 to 5, wherein the powdery mixture additionally contains 0.2 to 2% by weight of platelet extract.

7. The plasma derivative according to anyone of the claims 2 to 6, wherein the powdery mixture consists of

| 20 to 50% | by weight of thrombin, |
| 5 to 40% | by weight of prothrombin, |
| 8 to 70% | by weight of at least one agent selected from albumin, globulin, and fibrinogen, |
| 3 to 12% | by weight of water-soluble collagen, |
| 0.5 to 1.2% | by weight of platelet extract, and, |
| 2 to 6% | by weight of fibrinolysis inhibitor. |

8. The plasma derivative according to anyone of the claims 2 to 7, wherein the powdery mixture additionally contains 1 to 10 units of factor VIII and/or factor IX per 1 g of powdery mixture.

9. The plasma derivative according to anyone of the claims 2 to 8, wherein the powdery mixture additionally is enriched with bactericides and/or antibiotics, such as penicillins, aureomycins, streptomycins, and the like.

10. The plasma derivative according to anyone of the claims 2 to 9, wherein the powdery mixture has an average particle size ranging from 0,1 μm to 5 μm.

11. The plasma derivative according to anyone of the claims 2 to 10, wherein a solvent serving as a propellant is employed and wherein said solvent boils below 10°C at atmospheric pressure (100 kPa).

12. The plasma derivative according to anyone of the claims 1 to 11, wherein the solvent serving as propellant is an aliphatic halogenated hydrocarbon.

13. The plasma derivative according to claim 12, wherein the aliphatic halogenated hydrocarbon is tetrafluorodichloro-ethane ($C_2F_4Cl_2$) or difluorodichloro-methane ($CF_2Cl_2$).

14. A method of preparing an enriched plasma derivative in form of a biochemical substrate useful for accelerated hemostasis and optimized control of wound sealing, the composition thereof is selected with regard to an optimized activation of the exogenic and/or endogenic coagulation system and under consideration of a multiplicity of physiological and, if applicable, pathological aspects, characterized in that the constituents thereof, namely thrombin, which may partly be replaced by components of the prothrombin complex, fibrinolysis inhibitors and at least one desiccating and stabilizing agent selected from a group including albumin, globulin and fibrinogen, and optional admixtures of blood platelet extracts, antibiotics, and the like, which constituents exclusively are provided in powdery state, and a propellant are prepared in the form of a spray.

15. The method according to claim 14, wherein a powdery admixture containing

| 15 to 60% | by weight of thrombin, |
| 5 to 80% | by weight of at least one desiccating and stabilizing agent selected from albumin, globulin, and fibrinogen, and |
| 1 to 10% | by weight of a fibrinolysis inhibitor |

10

(respectively based on the weight of the powder) is suspended in a low-boiling anhydrous solvent serving as propellant.

16. The method according to claim 15, wherein a part of said thrombin is replaced by prothrombin.

17. The method according to claim 15 or 16, wherein 2 to 24% by weight of powdery, water-soluble collagen are added to said powdery mixture.

18. The method according to anyone of the claims 15 to 17, wherein 0.2 to 2% by weight of platelet extract are added to said powdery mixture.

19. The method according to anyone of the claims 15 to 18, wherein a powdery mixture consisting of

| | |
|---|---|
| 20 to 50% | by weight of thrombin, |
| 5 to 40% | by weight of prothrombin, |
| 8 to 70% | by weight of an agent selected from albumin, globulin, and fibrinogen, |
| 3 to 12% | by weight of water-soluble collagen, |
| 0.5 to 1.2% | by weight of platelet extract and |
| 2 to 6% | by weight of fibrinolysis inhibitor |

is suspended in said propellant.

20. The method according to anyone of the claims 15 to 19, wherein 1 to 10 units of factor VIII and/or factor IX per 1 g of powdery mixture, and/or bactericides and/or antibiotics, such as penicillins, aureomycins, streptomycins, and the like are added to said powdery mixture.

21. The method according to anyone of the claims 14 to 20, wherein an aliphatic halogenated hydrocarbon is used as said propellant.

22. The method according to claim 21, wherein tetrafluorodichloro-ethane ($C_2F_4Cl_2$) or difluorodichloromethane ($CF_2Cl_2$) is used as said aliphatic halogenated hydrocarbon.

## Revendications

1. Dérivé enrichi du plasma
sous forme d'un substrat biochimique destiné à l'hémostase accélérée et à la régulation optimale de la fermeture des plaies, dont la composition est choisie en vue d'une optimisation de l'activité du système de coagulation exogène et/ou endogène en tenant compte d'un grand nombre de points de vue physiologiques et éventuellement pathologiques,
parmi les constituants principaux duquel figurent la thrombine, qui peut être remplacée en partie par des constituants du complexe de prothrombine, des inhibiteurs de fibrinolyse et au moins un agent de siccité et de stabilisation choisi dans le groupe constitué de l'albumine, d'une globuline et du fibrinogène, et qui peut contenir, le cas échéant, en plus, des mélanges d'extraits de plaquettes sanguines, d'antibiotiques et de substances similaires,
dont les constituants principaux se présentent exclusivement à l'état pulvérulent, et
qui est conditionné en spray.

2. Dérivé enrichi du plasma suivant la revendication 1, pour favoriser la fermeture des plaies et pour les recouvrir, à base de dérivés du plasma humain ayant une teneur en thrombine et en inhibiteur de fibrinolyse, caractérisé en ce que un mélange de poudres contenant

| | |
|---|---|
| de 15 à 60% | en poids de thrombine, |
| de 5 à 80% | en poids d'agent de siccité et de stabilisation, à savoir de l'albumine, une globuline et/ou du fibrinogène, et |
| de 1 à 10% | en poids d'un inhibiteur de fibrinolyse |

(chacun rapporté au poids de la poudre) est en suspension dans un solvant à bas point d'ébullition, anhydre et servant d'agent propulseur.

3. Dérivé du plasma suivant la revendication 2, caractérisé en ce que une partie de la thrombine est remplacée par de la prothrombine.

4. Dérivé du plasma suivant la revendication 3, caractérisé en ce que le mélange de poudres contient de 5 à 40% en poids de prothrombine.

5. Dérivé du plasma suivant l'une des revendications 2 à 4, caractérisé en ce que le mélange de poudres contient, en outre, de 2 à 24% en poids de collagène pulvérulent soluble dans l'eau.

6. Dérivé du plasma suivant l'une des revendications 2 à 5, caractérisé en ce que le mélange de poudres contient, en outre, de 0,2 à 2% en poids d'extrait de plaquettes.

7. Dérivé du plasma suivant l'une des revendications 2 à 6, caractérisé en ce que le mélange de poudre est constitué

| | |
|---|---|
| de 20 à 50% | en poids de thrombine, |
| de 5 à 40% | en poids de prothrombine, |
| de 8 à 70% | en poids d'albumine, d'une globuline et/ou de fibrinogène, |

11

de 3 à 12% en poids de collagène soluble dans l'eau,
de 0,5 à 1,2%en poids d'extrait de plaquettes et
de 2 à 6% en poids d'inhibiteur de fibrinolyse.

8. Dérivé du plasma suivant l'une des revendications 2 à 7, caractérisé en ce que le mélange de poudres est enrichi, en outre, de 1 à 10 unités de facteur VIII et/ou de facteur IX par gramme de mélange de poudres.

9. Dérivé du plasma suivant l'une des revendications 2 à 8, caractérisé en ce que le mélange de poudres est enrichi, en outre, d'agents bactéricides et/ou d'antibiotiques comme des pénicillines, des auréomycines, des streptomycines et des substances similaires.

10. Dérivé du plasma suivant l'une des revendications 2 à 9, caractérisé en ce que le mélange de poudres présente une granulométrie moyenne comprise entre 0,1 et 5 microns.

11. Dérivé du plasma suivant l'une des revendications 2 à 10, caractérisé en ce que le solvant servant d'agent propulseur bout sous la pression atmosphérique (100 kPa) en-dessous de 10° C.

12. Dérivé du plasma suivant l'une des revendications 1 à 11, caractérisé en ce que le solvant servant d'agent propulseur est un hydrocarbure halogéné aliphatique.

13. Dérivé du plasma suivant la revendication 12, caractérisé en ce que l'hydrocarbure halogéné aliphatique est le tétrafluorodichloroéthane ($C_2F_4Cl_2$) ou le difluorodichlorométhane ($CF_2Cl_2$).

14. Procédé de préparation d'un dérivé enrichi du plasma, sous forme d'un substrat biochimique destiné à l'hémostase accélérée et à la régulation optimale de la fermeture des plaies, dont la composition est choisie en vue d'une optimisation de l'activité du système de coagulation exogène et/ou endogène en tenant compte d'un grand nombre de points de vue physiologiques et éventuellement pathologiques, caractérisé en ce que les constituants présents exclusivement à l'état pulvérulent, à savoir la thrombine, qui peut être remplacée partiellement par des constituants du complexe de prothrombine, des inhibiteurs de fibrinolyse et au moins un agent de siccité et de stabilisation choisi dans le groupe de l'albumine, d'une globuline et du fibrinogène, ainsi que, le cas échéant, des mélanges existants d'extraits de plaquettes sanguines, d'antibiotiques et de substances similaires, sont conditionnés ensemble avec un agent propulseur sous la forme d'un spray.

15. Procédé suivant la revendication 15, caractérisé en ce qu'il consiste à mettre un mélange de poudres contenant

de 15 à 60% en poids de thrombine,
de 5 à 80% en poids d'un agent de siccité et de stabilisation, à savoir l'albumine, une globuline et/ou du fibrinogène, et
de 1 à 10% en poids d'inhibiteur de fibrinolyse

(chacun rapporté au mélange de poudres) en suspension dans un solvant à bas point d'ébullition, anhydre et servant d'agent propulseur.

16. Procédé suivant la revendication 15, caractérisé en ce qu'il consiste à remplacer une partie de la thrombine par de la prothrombine.

17. Procédé suivant la revendication 15 ou 16, caractérisé en ce qu'il consiste à ajouter au mélange de poudres, en outre, de 2 à 24% en poids de collagène pulvérulent soluble dans l'eau.

18. Procédé suivant l'une des revendications 15 à 17, caractérisé en ce qu'il consiste à ajouter au mélange de poudres, en outre, de 0,2 à 2% en poids d'extrait de plaquettes.

19. Procédé suivant l'une des revendications 15 à 18, caractérisé en ce qu'il consiste à mettre en suspension, dans l'agent propulseur, un mélange de poudres constitué

de 20 à 50% en poids de thrombine,
de 5 à 40% en poids de prothrombine,
de 8 à 70% en poids d'albumine, d'une globuline et/ou de fibrinogène,
de 3 à 12% en poids de collagène soluble dans l'eau,
de 0,5 à 1,2%en poids d'extrait de plaquettes et
de 2 à 6% en poids d'inhibiteur de fibrinolyse.

20. Procédé suivant l'une des revendications 15 à 19, caractérisé en ce qu'il consiste à enrichir le mélange de poudres, en outre, de 1 à 10 unités de facteur VIII et/ou de facteur IX par gramme de mélange de poudres et/ou à l'enrichir, en outre, d'agents bactéricides et/ou d'antibiotiques, comme des pénicillines, des auréomycines, des streptomycines et des substances semblables.

21. Procédé suivant l'une des revendications 14 à 20, caractérisé en ce qu'il consiste à se servir, comme agent propulseur, d'un hydrocarbure halogéné aliphatique.

22. Procédé suivant la revendication 21, caractérisé en ce qu'il consiste à se servir, comme hydrocarbure halogéné aliphatique, de tétrafluorodichloroéthane ($C_2F_4Cl_2$) ou de difluorodichlorométhane ($CF_2Cl_2$).